# EUROPÄISCHE PATENTSCHRIFT

(11) **EP 2 099 738 B1**
(45) Veröffentlichungstag und Bekanntmachung des Hinweises auf die Patenterteilung: **01.02.2017**
(21) Anmeldenummer: 07765119.8
(22) Anmeldetag: 07.07.2007
(51) Int. Cl.: C07C 213/02

(54) **VERFAHREN ZUR HERSTELLUNG VON POLYETHERAMINEN**
PROCESS FOR PREPARING POLYETHERAMINES
PROCÉDÉ DE FABRICATION DE POLYÉTHERAMINES

(30) Priorität: 06.12.2006 DE 102006057457
(43) Veröffentlichungstag der Anmeldung: 16.09.2009
(73) Patentinhaber: Clariant International Ltd, 4132 Muttenz (CH)
(72) Erfinder: BÜHRING, Dirk, 84489 Burghausen (DE); GALLAS, Andreas, 84567 Erlbach (DE); RAAB, Klaus, 84508 Burgkirchen (DE); SCHERL, Franz Xaver, 84508 Burgkirchen (DE); WACHSEN, Olaf, 84518 Garching (DE)
(74) Vertreter: Mikulecky, Klaus
(86) Internationale Anmeldenummer: PCT/EP2007/006037
(87) Internationale Veröffentlichungsnummer: WO 2008/067857

(56) Entgegenhaltungen:
- EP-A- 0 436 235
- WO-A-2007/000236

## Beschreibung

Die vorliegende Erfindung betrifft ein Verfahren zur Herstellung von Polyetheraminen unter Verwendung von Cobalt-Katalysatoren.

Polyetheramine enthalten mindestens eine Polyalkylenglykolgruppe, die deren Eigenschaften maßgeblich beeinflusst. Bei hohem Anteil an Polyethylenglykoleinheiten erhält man wasserlösliche und bei hohem Anteil von Polypropylenglykoleinheiten wasserunlösliche Polyetheramine. Außerdem lassen sich durch die Veränderung der Molmassen der Polyalkylenglykole Schmelzpunkt und Viskosität der Polyetheramine beeinflussen. Durch die Auswahl geeigneter Startalkohole für die Alkoxylierung lassen sich tensidische Eigenschaften erzielen. Außerdem kann man mit mehrwertigen Alkoholen verzweigte Polyetheramine aufbauen, die dann nach der Aminolyse zu mehrwertigen Aminen führen. Damit ergeben sich vielfältige Möglichkeiten durch die Wahl geeigneter Polyalkylenglykole die Eigenschaften eines darauf basierenden Polyetheramins gezielt zu beeinflussen.

Der Stand der Technik offenbart verschiedene Verfahren zur Herstellung von Polyetheraminen.

DE-A-16 43 426 beschreibt ein Verfahren zur Herstellung von Polyoxyalkylenaminen, ausgehend von den entsprechenden Alkoholen unter Verwendung eines Nickel-Kupfer-Chrom-Katalysators, der 60 bis 85 Mol-% Nickel, 14 bis 37 Mol-% Kupfer und 1 bis 5 Mol-% Chrom enthält.

US-4 618 717 beschreibt ein Verfahren zur Herstellung von primären Aminen, ausgehend von Oxyethylenglykol-Monoalkylethern unter Verwendung eines Katalysators, der 50 bis 90 Gew.-% Nickel, 10 bis 15 Gew.-% Kupfer und 0,5 bis 5 Gew.-% der Elemente Chrom, Eisen, Titan, Thorium, Zirkon, Mangan, Magnesium oder Zink enthält.

WO-03/076386 lehrt Katalysatoren, deren katalytisch aktive Masse vor der Behandlung mit Wasserstoff 22 bis 40 Gew.-% sauerstoffhaltige Verbindungen des Zirkoniums, berechnet als ZrO₂, 1 bis 30 Gew.-% sauerstoffhaltige Verbindungen des Kupfers, berechnet als CuO, 15 bis 50 Gew.-% sauerstoffhaltige Verbindungen des Nickels, berechnet als NiO, wobei das Molverhältnis von Nickel zu Kupfer größer 1 ist, 15 bis 50 Gew.-% sauerstoffhaltige Verbindungen des Kobalts, berechnet als CoO, und weniger als 1 Gew.-% Alkalimetall, berechnet als Alkalimetalloxid, enthält, und Verfahren zur Herstellung von Aminen durch Umsetzung von primären oder sekundären Alkoholen, Aldehyden oder Ketonen bei erhöhter Temperatur und erhöhtem Druck mit Wasserstoff und Stickstoffverbindungen, ausgewählt aus der Gruppe Ammoniak, primäre und sekundäre Amine, in Gegenwart dieser Katalysatoren.

WO-2007/000236 lehrt ein Verfahren zur Herstellung von Aminen der Formel (1), worin R² einen organischen Rest darstellt, der zwischen 2 und 600 Alkoxygruppen umfasst, und R¹ und R³ gleich oder verschieden sind und Wasserstoff oder einen organischen Rest mit 1 bis 400 Kohlenstoffatomen darstellen, indem man eine Verbindung der Formel (2) mit einer Verbindung der Formel (3) in Gegenwart von Wasserstoff mit einem Katalysator in Verbindung bringt, der metallhaltig ist und dessen Metallgehalt zu mindestens 80 Gew.-% aus Cobalt besteht.

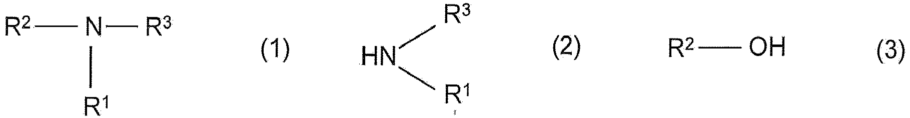

US-5 352 835 beschreibt einen Trägerkatalysator zur Aminolyse, der zur Umsetzung von Polyoxyalkylenglykolen zu den entsprechenden Aminen verwendet wird. Hierbei besteht der Katalysator aus 15 bis 30 Gew.-% Nickel, 3 bis 20 Gew.-% Kupfer, 0,5 bis 1 Gew.-% Molybdän und mindestens 50 Gew.-% γ-Aluminiumoxid, welches als Trägermaterial dient.

US-4 766 245 beschreibt ein Verfahren zur Herstellung von Polyoxyalkylenpolyaminen ausgehend von den entsprechenden Alkoholen unter Verwendung eines Raney-Nickel/Aluminium-Katalysators der zu 60 bis 75 % aus Nickel und zu 40 bis 25 % aus Aluminium besteht.

DE-A-36 08 716 beschreibt ein Verfahren zur Herstellung von Polyoxyalkylenpolyaminen, ausgehend von den entsprechenden Alkoholen unter Verwendung eines Raney-Nickel- oder Raney-Nickel/Aluminium-Katalysators, der zusätzlich noch 0,2 bis 5 % Molybdän enthält.

US-5 003 107 beschreibt ein Verfahren zur reduktiven Aminierung von Polyoxytetramethylenglykolen unter Verwendung eines Katalysators, der 70 bis 75 Gew.-% Nickel, 20 bis 25 Gew.-% Kupfer, 0,5 bis 5 Gew.-% Chrom und 1 bis 5 Gew.-% Molybdän enthält.

DE-A-44 28 004 beschreibt ein Verfahren zur Herstellung von Aminen ausgehend von Alkoholen, bei dem der Katalysator 20 bis 85 % Zr, 1 bis 30 % Cu, 30 bis 70 % Ni, 0,1 bis 5 % Mo, 0 bis 10 % Aluminium und/oder Mangan, jeweils als Oxid berechnet, enthält.

US-A-2003/0139289 beschreibt ein verbessertes Verfahren zur Herstellung von Aminen ausgehend von Alkoholen, Aldehyden oder Ketonen mittels eines Katalysators, der neben Nickel, Kupfer und Chrom noch Zinn enthält.

DE-A-19 53 263 beschreibt ein Verfahren zur Herstellung von Aminen ausgehend von Alkoholen mittels eines Katalysators. Der Metallanteil des Katalysators besteht zu 70 bis 95 % aus Co und Ni und zu 5 bis 30 % aus Kupfer. Hierbei reicht das Gewichtsverhältnis von Co zu Ni von 4:1 bis 1:4.

DE-A-102 11 101 beschreibt ein Verfahren zur Herstellung von Aminen ausgehend von Alkoholen oder Aldehyden, bei dem der Katalysator 22 bis 40 % Zr, 1 bis 30 % Cu, 15 bis 50 % Ni, 15 bis 50 % Co, jeweils als Oxid berechnet, enthält.

DE-A-15 70 542 offenbart ein Verfahren zur Herstellung von Polyetheraminen, dadurch gekennzeichnet, dass man Polyether der Formel I oder II

HO-R-(OR)ₙ-OH (I)

Z[(OR)ₙ-OH]ₘ (II)

worin R einen aliphatischen Rest mit 2 bis 4 C-Atomen bedeutet, Z für einen zweibis sechswertigen aliphatischen, araliphatischen, aromatischen oder alicyclischen Rest steht, der ein- oder mehrfach durch Ether- oder Aminogruppen, Carbonamid-, Urethan- oder Harnstoffgruppen unterbrochen sein kann, n ganze Zahlen zwischen 1 und 50 und m = 2 bis 6 je nach der Wertigkeit von Z darstellt, in Gegenwart von Wasser und Wasserstoff an einem hauptsächlich aus einem Element der 8. Nebengruppe bestehenden Hydrierungs-Dehydrierungskontakt mit Ammoniak bei 200 bis 280°C, vorzugsweise 220 bis 250°C, unter Druck umsetzt.

Überraschenderweise wurde nun gefunden, dass Polyetheramine bzw. Polyoxyalkylenamine, insbesondere primäre Polyetheramine bzw. Polyoxyalkylenamine, aus den entsprechenden Alkoholen und Ammoniak bzw. Aminen in Gegenwart von Wasserstoff mit hohen Umsetzungsgraden und Selektivitäten hergestellt werden können, indem man einen heterogenen Katalysator verwendet, dessen Metallgehalt, bezogen auf den trockenen, reduzierten Katalysator ohne eventuell vorhandenes Trägermaterial, entweder zu mindestens 80 Masse-% aus Cobalt oder zu mindestens 80 Masse-% aus Metallen der Gruppe Cobalt und Aluminium besteht, und der weniger als 5 Masse-% Kupfer umfasst.

Es können hierbei sowohl Trägerkatalysatoren, die außer dem katalytisch aktiven Metallanteil noch Trägermaterial enthalten, als auch Metallkatalysatoren ohne zusätzliches Trägermaterial, wie beispielsweise die sogenannten "Raney-Katalysatoren" verwendet werden.

Das Verfahren ist sowohl zur kontinuierlichen als auch zur diskontinuierlichen Herstellung von Polyetheraminen geeignet.

Gegenstand der Erfindung ist somit ein Verfahren zur Herstellung von Polyetheraminen der Formel 1

R²(NR¹R³)ₙ (1)

worin n eine Zahl von 1 bis 20 ist, R² einen organischen Rest darstellt, der zwischen 2 und 600 Oxalkylengruppen enthält, und R¹ und R³ gleich oder verschieden sind und Wasserstoff oder einen organischen Rest mit 1 bis 400 Kohlenstoffatomen darstellen, indem man eine Verbindung der Formel 2 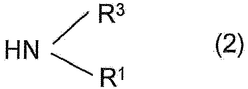 mit einer Verbindung der Formel 3

R²(OH)ₙ (3)

in Gegenwart von Wasserstoff mit einem Katalysator in Verbindung bringt, der metallhaltig ist und dessen Metallgehalt, bezogen auf den trockenen, reduzierten Katalysator ohne eventuell vorhandenes Trägermaterial, entweder zu mindestens 95 Masse-% aus Cobalt oder im Falle der Raney-Katalysatoren zu mindestens 80 Masse-% aus Metallen der Gruppe Cobalt und Aluminium besteht, wobei der Katalysator weniger als 5 Masse-% Kupfer enthält.

Der für das erfindungsgemäße Verfahren verwendete Katalysator enthält, bezogen auf den trockenen, reduzierten Katalysator ohne eventuell vorhandenes Trägermaterial, entweder mindestens 95 Masse-% Cobalt oder im Falle der Raney-Katalysatoren vorzugsweise mindestens 85 Masse-%, insbesondere mindestens 90 Masse-%, speziell mindestens 95 Masse-% Metalle aus der Gruppe Cobalt und Aluminium. Im Falle der Raney-Katalysatoren ist bevorzugt, dass der Cobaltgehalt höher liegt als der Aluminiumgehalt.

Der Katalysator kann neben Cobalt oder Cobalt und Aluminium in untergeordneten Anteilen noch andere Metalle wie Nickel, Chrom, Eisen, Thorium, Mangan, Molybdän, Zink, Lithium und/oder Zinn enthalten. Diese Anteile liegen, bezogen auf den trockenen, reduzierten Katalysator ohne eventuell vorhandenes Trägermaterial, unter 20 Masse-%, vorzugsweise unter 15 Masse-%, insbesondere unter 10 Masse-% und speziell unter 5 Masse-%.

Es wurde gefunden, dass ein niedriger Kupfergehalt des Katalysators insbesondere bei der Herstellung primärer Polyetheramine vorteilhaft ist. Dies trifft auf Raney-Katalysatoren und auf Träger-Katalysatoren zu. Der Kupfergehalt des Katalysators, bezogen auf den trockenen, reduzierten Katalysator ohne eventuell vorhandenes Trägermaterial, soll daher unter 5 Masse-%, vorzugsweise unter 4 Masse-%, insbesondere unter 2 Masse-%, speziell zwischen 1 Masse-% und 0,1 Masse-% liegen. In einer besonders bevorzugten Ausführungsform werden Katalysatoren mit Kupfergehalten, wobei der Kupfergehalt bezogen ist auf den trockenen, reduzierten Katalysator ohne eventuell vorhandenes Trägermaterial, von unter 5 Masse-%, vorzugsweise unter 4 Masse-%, insbesondere unter 2 Masse-%, speziell zwischen 1 Masse-% und 0,1 Masse-% für die Herstellung von primären Polyetheraminen verwendet, in denen R¹ und R³ gleichzeitig Wasserstoff bedeuten, und in denen n gleich eins oder gleich zwei ist.

Der Katalysator kann ein geträgerter oder ein ungeträgerter Katalysator sein.

Ist der Katalysator ungeträgert, so handelt es sich vorzugsweise um einen Katalysator vom Typ Raney-Katalysator. Raney-Katalysatoren, wie sie beim erfindungsgemäßen Verfahren eingesetzt werden, bestehen im trockenen Zustand hauptsächlich aus Cobalt und Aluminium, wobei die Summe aus Cobalt-und Aluminium-Gehalt für den Einsatz beim erfindungsgemäßen Verfahren mindestens 80 Masse-%, vorzugsweise mindestens 85 Masse-%, insbesondere mindestens 90 Masse-%, speziell mindestens 95 Masse-% bezogen auf den Metallanteil des trockenen Katalysators beträgt. Raney-Katalysatoren werden wegen ihrer pyrophoren Eigenschaften oftmals unter Wasser gelagert und die Entfernung des Wassers unter Schutzgas vor Einsatz bei der Herstellung von Polyetheraminen ist meist vorteilhaft. Im Falle von pulverförmigen Raney-Katalysatoren kann die mittlere Teilchengröße zwischen beispielsweise 20 und 200 Mikrometer liegen. Im Falle von Festbett-Raney-Katalysatoren kann die äußere Form beispielsweise unregelmäßig, kugelförmig oder hohlkugelförmig sein mit Abmessungen im Bereich von 1 bis 6 mm. Unter den Raney-Katalysatoren sind die hohlkugelförmigen Raney-Katalysatoren wegen ihrer hohen Porosität bevorzugt. Im wasserfreien, aktivierten Zustand bestehen die Raney-Katalysatoren bevorzugt aus 40 bis 99 Masse-% Cobalt und 60 bis 1 Masse-% Aluminium, insbesondere aus 55 bis 98 Masse-% Cobalt und 45 bis 2 Masse-% Aluminium.

Ist der Katalysator geträgert, so kann der Katalysator eine beliebige geometrische Form haben, beispielsweise die Form von Kugeln, Tabletten, Stäbchen oder Zylindern in regelmäßiger oder unregelmäßiger Ausführung. Die Abmessung des geträgerten Katalysators für Festbettreaktoren liegt im Allgemeinen zwischen 1 und 8 mm.

Geträgerte Katalysatoren in Pulverform besitzen Teilchengrößen meist unter 200 Mikrometer und werden beispielsweise in Rührreaktoren oder Schlaufenreaktoren eingesetzt.

Als Träger eignen sich die bekannten Trägermaterialien wie Aluminiumoxid, Kieselsäure, amorphes Siliciumdioxid, Kieselgur, Alumosilikate, Silikate, Titandioxid, Titanate, Zirkondioxid, Magnesiumoxid, Calciumoxid, Mischungen der vorgenannten Oxide, Siliciumcarbid und Aktivkohle. Besonders geeignet sind Träger dieser Art mit einer spezifischen Oberfläche von 50 bis 300 m²/g (gemessen nach der BET-Methode) und einem mittleren Porenradius von 5 nm bis 200 nm (gemessen mit Quecksilber-Porosimetrie), vor allem Aluminiumoxid, amorphes Siliciumdioxid, Kieselsäure, Kieselgur (SiO₂) und SiO₂-Al₂O₃-Gemische. Makroporöse Träger mit großem Porenradius zwischen 10 nm und 100 nm werden bevorzugt, speziell solche mit Aluminiumoxid als Trägermaterial.

Die Trägerkatalysatoren werden bevorzugt im reduzierten und damit aktivierten Zustand im erfindungsgemäßen Verfahren eingesetzt. Die Trägerkatalysatoren des erfindungsgemäßen Verfahrens bestehen zu mindestens 80%, vorzugsweise zu mindestens 85 Masse-%, insbesondere zu mindestens 90 Masse-%, speziell zu mindestens 95 Masse-% aus Cobalt, wobei diese Gehaltsangabe sich nur auf den Metallanteil des reduzierten Katalysators bezieht unter Ausschluss des Anteils an Trägermaterial.

R² enthält 2 bis 600 Oxalkylengruppen. Unter Oxalkylengruppen wird vorliegend eine Einheit der Formel -(A-O)- verstanden, worin A für eine C₂- bis C₄-Alkylengruppe steht. 2 bis 600 Oxalkylengruppen bedeuten damit eine Struktureinheit der Formel -(A-O)ₙ- mit n = 2 bis 600.

In der durch (A-O)ₙ wiedergegebenen Oxalkylenkette bedeutet A vorzugsweise einen Ethylenrest -CH₂-CH₂- oder einen Propylenrest -CH₂-CH(CH₃)-, insbesondere einen Ethylenrest. Die Gesamtzahl von Oxalkyleneinheiten liegt vorzugsweise zwischen 5 und 300, insbesondere zwischen 8 und 200. Bei der Oxalkylenkette kann es sich um eine Blockpolymerkette handeln, die alternierende Blöcke verschiedener Oxalkyleneinheiten, vorzugsweise Oxethylen-und Oxpropyleneinheiten, aufweist. Es kann sich dabei auch um eine Kette mit statistischer Abfolge der Oxalkyleneinheiten, um eine Kette mit Kettenabschnitten aus Blöcken verschiedener Oxalkyleneinheiten und mit Kettenabschnitten mit statistischer Abfolge der Oxalkyleneinheiten oder um ein Homopolymer handeln.

In einer bevorzugten Ausführungsform steht -(A-O)ₙ- für eine Oxalkylenkette der Formel 4

- (CH(CH₃) - CH₂ - O)ₐ - (CH₂ - CH₂ - O)_{b} - (CH₂ - CH(CH₃) - O)_{c} - (4)

worin
- a: eine Zahl von 0 bis 300, vorzugsweise 2 bis 80
- b: eine Zahl von 3 bis 300, vorzugsweise 3 bis 200
- c: eine Zahl von 0 bis 300, vorzugsweise 2 bis 80 bedeuten.

R¹ und R³ sind gleich oder verschieden und stehen für Wasserstoff oder einen organischen Rest mit 1 bis 400 Kohlenstoffatomen. R¹ und R³ können neben Kohlenstoff und Wasserstoff auch Heteroatome wie Sauerstoff, Stickstoff, Phosphor oder Schwefel enthalten.

In einer bevorzugten Ausführungsform sind R¹ und R³ Wasserstoff, ein Alkylrest mit 1 bis 50 Kohlenstoffatomen, ein Alkenylrest mit 2 bis 50 Kohlenstoffatomen, ein Arylrest mit 6 bis 50 Kohlenstoffatomen, oder ein Alkylarylrest mit 7 bis 50 Kohlenstoffatomen.

Insbesondere ist R¹ = R³= H_{.}

In einer weiteren bevorzugten Ausführungsform entsprechen R¹ und R³ der gleichen Definition wie R².

In einer weiteren bevorzugten Ausführungsform enthalten R¹ und R³ Aminogruppen. Vorzugsweise entsprechen R¹ und R³ dann der Formel 5 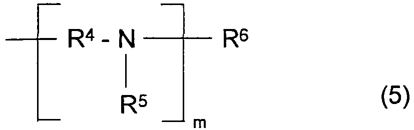 worin R⁴ eine zweiwertige Kohlenwasserstoffgruppe mit 1 bis 50 Kohlenstoffatomen und R⁵ und R⁶ jeweils Wasserstoff oder eine einwertige Kohlenwasserstoffgruppe mit 1 bis 50 Kohlenstoffatomen sein können, wobei jedes von R⁴, R⁵ und R⁶ 1 bis 200 Oxalkylengruppen umfassen kann und auch Heteroatome wie Sauerstoff, Stickstoff, Phosphor oder Schwefel enthalten können (im Grunde wie R³), und m für eine Zahl von 1 bis 10 steht.

Wenn R² für alkoxyliertes Methanol steht, so kann das Produkt des erfindungsgemäßen Verfahrens beispielsweise folgende Strukturen aufweisen:

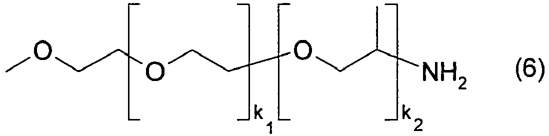

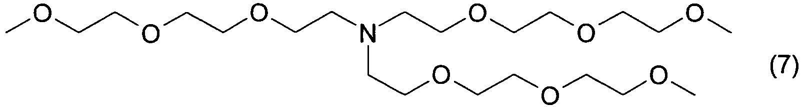

Im Falle eines alkoxylierten Alkyl-Alkohols ergibt sich folgende Struktur:

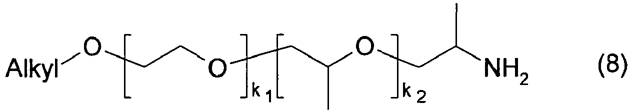

Bei Verwendung von Polyalkylenglykolen (Diolen) kommt man zu folgenden Strukturen: 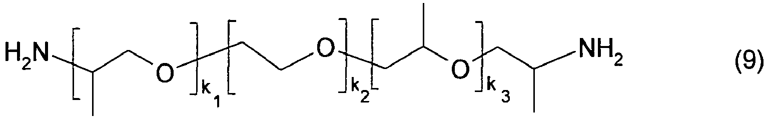 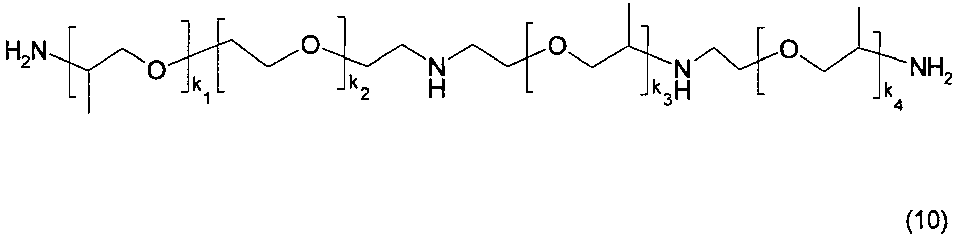 worin k₁, k₂, k₃ und k₄ ganze Zahlen darstellen, die in der Summe bis zu 600 ergeben.

In einer weiteren bevorzugten Ausführungsform stehen n für 1, 2, 3 oder 4 und R² für einen organischen Rest, der zwischen 5 und 300 Oxalkylengruppen enthält.

In einer weiteren bevorzugten Ausführungsform stehen n für eine ganze Zahl von 5 bis 20 und R² für einen organischen Rest, der 2 bis 300 Oxalkylengruppen enthält.

Wenn R² für alkoxyliertes Glycerin steht, so kann das Produkt des erfindungsgemäßen Verfahrens beispielsweise folgende Struktur aufweisen: 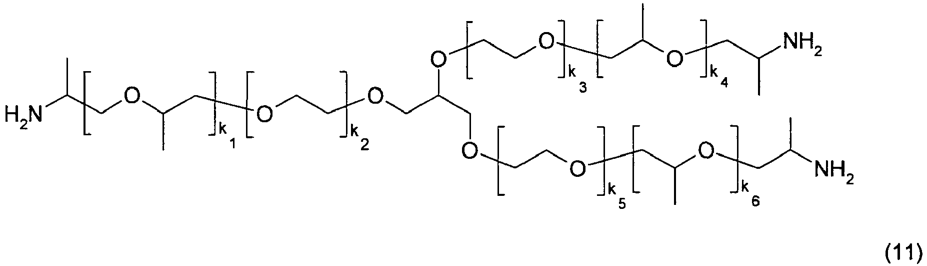 worin die Indices kₙ ganze Zahlen sind, die in der Summe bis zu 600 ergeben.

Wenn R² für alkoxyliertes Octylamin steht, so kann das Produkt des erfindungsgemäßen Verfahrens beispielsweise folgende Struktur aufweisen: 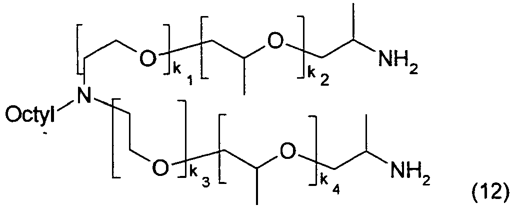 worin die Indices kₙ ganze Zahlen sind, die in der Summe bis zu 600 ergeben.

Erfindungsgemäße Polyetheramine sind ein- oder mehrwertige Amine, die verzweigt, unverzweigt oder cyclisch, gesättigt oder ungesättigt sein können. Es kann sich dabei um primäre, sekundäre oder tertiäre Amine handeln. Bevorzugt sind primäre Amine mit R¹ = R³ = H.

Erfindungsgemäße Polyetheramine sind beispielsweise
- einwertige Amine, wie beispielsweise
   Alkylpolyalkylenglykolamine wie z.B. Methyltriethylenglykolamin, Bis(methyltriethylenglykol)amin, Butyltriethylenglykolamin, Laurylpolypropylenglykolamin, Methyltripropylenglykolamin, Phenolpolypropylenglykolamin, iso-Tridecylpolypropylenglykolamin, Bis(methyltripropylenglykol)amin, N-Methyl-Methylpolypropylenglykolamin, Methylpolypropylenglykolamin, Bis(methylpolypropylenglykol)amin, Tris(methyldiglykol)amin, Methylpolyalkylenglykolamin mit statistischer oder blockartiger Verteilung der Oxethylen- und Oxpropylen-Einheiten,
- zweiwertige Amine, wie beispielsweise
   Triethylenglykoldiamin, Tripropylenglykoldiamin, Polyethylenglykoldiamine, Polypropylenglykoldiamin, Polylalkylenglykoldiamin mit statistischer oder blockartiger Verteilung der Oxethylen- und Oxpropylen-Einheiten, Butandiolpolyalkylenglykoldiamin, Resorcinpolyalkylenglykoldiamin,
- dreiwertige Amine, wie beispielsweise
   Glycerinpolyalkylenglykoltriamin mit statistischer oder blockartiger Verteilung der Oxethylen- und Oxpropylen-Einheiten, Bis(triethylenglykolamin)amin, Bis(polyalkylenglykolamin)amin,
- vierwertige Amine, wie beispielsweise
   Pentaerythrolpolyalkylenglykoltetraamin mit statistischer oder blockartiger Verteilung Oxethylen- und Oxpropylen-Einheiten, N,N'-Bis(polypropylenglykolamin)-polyalkylenglykoldiamin.

Als Aminierungsmittel der Formel (2) bei der Aminierung der Polyetheralkohole der Formel (3) zu den Polyetheraminen der Formel (1) nach dem erfindungsgemäßen Verfahren können sowohl Ammoniak als auch primäre oder sekundäre, aliphatische oder cycloaliphatische oder aromatische Amine eingesetzt werden.

Bei der Verwendung von Ammoniak als Aminierungsmittel wird zunächst ein primäres Polyetheramin gebildet. Bei entsprechenden Reaktionsbedingungen (Druck, Temperatur, Reaktionszeit, Katalysator) kann dies als Produkt isoliert werden, oder die Reaktion wird weitergeführt, so dass das gebildete primäre Polyetheramin mit weiterem Alkohol zum entsprechenden sekundären oder auch tertiären Polyetheramin weiterreagiert.

Neben Ammoniak können beispielsweise folgende Amine als Aminierungsmittel zum Einsatz kommen: Methylamin, Dimethylamin, Ethylamin, Diethylamin, n-Propylamin, Di-n-Propylamin, iso-Propylamin, Di-iso-Propylamin, Hexylamin, Cyclohexylamin, Anilin, Toluidin, Piperidin, Morpholin.

Das Aminierungsmittel kann bezüglich der zu aminierenden Hydroxyl-Gruppe in stöchiometrischen, unter- oder überstöchiometrischen Mengen eingesetzt werden. Wenn hohe Umsätze des Alkohols der Formel (3) erzielt werden sollen, wird das Aminierungsmittel wie beispielsweise Ammoniak vorzugsweise im molaren Überschuss eingesetzt und unumgesetztes Aminierungsmittel zurückgewonnen. Ebenso kann der zu verwendende Wasserstoffpartialdruck in einem weiten Bereich, von beispielsweise 1 bar bis 60 bar, insbesondere von 10 bar bis 40 bar variiert werden. Wasserstoff wird nicht stöchiometrisch verbraucht, ist aber für die Aufrechterhaltung der Katalysatoraktivität erforderlich.

Das erfindungsgemäße Verfahren wird vorzugsweise bei Temperaturen von 50 bis 250°C, insbesondere bei Temperaturen von 140°C bis 200°C durchgeführt. Kontinuierliche Aminolyseverfahren im Festbettreaktor werden bevorzugt bei Temperaturen von 140°C bis 200°C, besonders bevorzugt bei Temperaturen von 150°C bis 180°C beispielsweise in Rieselbettfahrweise oder Sumpffahrweise durchgeführt. Bei der Rieselbettfahrweise werden die Verbindungen der Formel (3) von oben in den senkrecht stehenden Festbettreaktor dosiert und bei Sumpffahrweise von unten.

Das erfindungsgemäße Verfahren wird vorzugsweise bei Gesamtdrucken von 1 bar bis 300 bar, im Falle von Ammoniak oder leicht flüchtigen Aminierungsmitteln vorzugsweise bei Gesamtdrucken von 30 bar bis 300 bar, insbesondere bei 50 bar bis 200 bar durchgeführt.

Die zu verwendende Katalysatormenge liegt vorzugsweise im Bereich von 0,5 bis größer 90 Masse-%, insbesondere 1 bis 80 Masse-%, speziell 2 bis 70 Masse-%, bezogen auf den verwendeten Alkohol der Formel (3). Mengen über 70 Masse-% werden insbesondere bei kontinuierlichen Verfahren im Festbettreaktor verwendet. In diskontinuierlichen Prozessen werden in der Regel geringere Katalysatormengen im Bereich von 2 bis 15 Masse-% pro Ansatz eingesetzt, der Katalysator zurückgewonnen und für sehr viele Ansätze in Folge wieder eingesetzt.

Die Aminierung kann kontinuierlich beispielsweise in Festbettreaktoren oder diskontinuierlich beispielsweise in Rührreaktoren oder Schlaufenreaktoren durchgeführt werden. Bei beiden Verfahren können die gasförmigen oder zum Teil auch überkritischen Reaktionskomponenten (Aminierungsmittel, Wasserstoff und eventuell Inertgase) im Kreis gefahren werden.

Das sich während der Reaktion bildende Wasser kann entweder im Reaktionsgemisch verbleiben oder auch gegebenenfalls entfernt werden, falls ansonsten die gewünschte Umsetzung hinsichtlich Reaktionsgeschwindigkeit, Katalysatorstandzeit, Ausbeute und/oder Selektivität leidet.

Die Aminierung wird bevorzugt ohne Lösungsmittel durchgeführt. Es können aber auch Lösungsmittel verwendet werden.

Der erhaltene Reaktionsaustrag wird vom Katalysator, von überschüssigem Aminierungsmittel, vom entstandenen Wasser, von Wasserstoff und gegebenenfalls Inertgasen befreit und das Polyetheramin je nach Bedarf entsprechend weiter gereinigt. Die entfernten Reaktionskomponenten können, eventuell nach entsprechender Aufarbeitung, dem Aminierungsprozess wieder zugeführt werden. Der Katalysator wird ohne Verlust an Aktivität oder Selektivität wieder eingesetzt.

### Beispiele

### Beispiel 1

In einen Schüttelautoklav mit 4,5 l Volumen, ausgestattet mit einem Katalysatorkorb, wurden 1,0 kg Methylpolyalkylenglykol mit einer mittleren Molmasse von 2000 g/mol, 200 g eines unter Stickstoff-Schutzgas vom Wasser befreiten Raney-Katalysators und 600 ml Ammoniak (flüssig) eingebracht. Der Raney-Katalysator hatte die Form von porösen Hohlkugeln mit 3 mm Außendurchmesser und bestand, bezogen auf den Metallgehalt im trockenen Zustand, aus 64 Masse-% Cobalt und 32 Masse-% Aluminium. Der Kupfergehalt betrug, bezogen auf den Metallgehalt im trockenen Zustand, 0,2 Masse-%. Anschließend wurden 30 bar Wasserstoff aufgepresst und der Autoklav verschlossen. Das Gemisch wurde unter Schütteln für 20 Stunden auf 180°C erwärmt. Der Gesamtdruck bei 180°C betrug etwa 160 bar. Nach dem Abkühlen auf 50°C wurde langsam entspannt um den Großteil des Ammoniaks und den Wasserstoff zu entfernen. Das verbleibende Reaktionsgemisch wurde im Vakuum von restlichen flüchtigen Anteilen wie Ammoniak und gebildetes Reaktionswasser befreit und filtriert. Über die Bestimmung der Aminzahl und der Hydroxylzahl konnte ein Umsetzungsgrad von 94 % zum entsprechenden Polyetheramin ermittelt werden. Die titrimetrisch ermittelte Selektivität für die primären Polyetheramine betrug 98 Äquivalent-% bezogen auf den Gesamtamingehalt.

Zur Ermittlung der Selektivitäten wurden die Verteilungen der primären, sekundären und tertiären Polyetheramine durch Maskierungsreaktionen mit Essigsäureanhydrid beziehungsweise mit Salicylaldehyd und jeweils nachfolgende Säure-Base-Titrationen ermittelt. Außerdem wurden die Polyetheramine sowie die mit Trichloracetylisocyanat derivatisierten Proben 1 H-NMR-spektroskopisch untersucht.

### Beispiel 2

In einen Schüttelautoklav mit 4,5 l Volumen, ausgestattet mit einem Katalysatorkorb, wurden 1,0 kg Methylpolyalkylenglykol mit einer mittleren Molmasse von 2000 g/mol, 130 g eines zuvor mit Wasserstoff reduzierten und aktivierten Trägerkatalysators und 600 ml Ammoniak (flüssig) eingebracht. Der reduzierte Trägerkatalysator hatte die Form von etwa 3 mm langen Stäbchen und bestand zu 90 Masse-% aus dem Trägermaterial Aluminiumoxid und zu 10 Masse-% aus Metallen, wobei der Anteil des Cobalts, bezogen auf den Metallgehalt ohne das Trägermaterial Aluminiumoxid, über 98 Masse-% und der Anteil des Kupfers, bezogen auf den Metallgehalt ohne das Trägermaterial Aluminiumoxid, 0,4 Masse-% betrug. Anschließend wurden 30 bar Wasserstoff aufgepresst und der Autoklav verschlossen. Das Gemisch wurde unter Schütteln für 5 Stunden auf 180°C erwärmt. Der Gesamtdruck bei 180°C betrug etwa 160 bar. Nach dem Abkühlen auf 50°C wurde langsam entspannt um den Großteil des Ammoniaks und den Wasserstoff zu entfernen. Das verbleibende Reaktionsgemisch wurde im Vakuum von restlichen flüchtigen Anteilen wie Ammoniak und gebildetes Reaktionswasser befreit und filtriert. Über die Bestimmung der Aminzahl und der Hydroxylzahl konnte ein Umsetzungsgrad von 97 % zum entsprechenden Polyetheramin ermittelt werden. Die titrimetrisch ermittelte Selektivität für die primären Polyetheramine betrug über 99 Äquivalent-% bezogen auf den Gesamtamingehalt.

### Beispiel 3

In einen Rührautoklav mit 2 I Volumen wurden 1,0 kg Methylpolyalkylenglykol mit einer mittleren Molmasse von 2000 g/mol, 200 g eines Trägerkatalysators, dessen Metallanteil, bezogen auf den trockenen, reduzierten Katalysator ohne das Trägermaterial SiO₂/MgO zu mehr als 95 Masse-% aus Cobalt und zu 0,8 Masse-% aus Kupfer bestand, und 540 ml Ammoniak (flüssig) dosiert. Anschließend wurden 30 bar Wasserstoff aufgepresst und der Autoklav verschlossen. Das Gemisch wurde unter Rühren für 7 Stunden auf 190°C erwärmt. Nach dem Abkühlen wurde langsam entspannt um den Ammoniak und den Wasserstoff zu entfernen. Das verbleibende Reaktionsgemisch wurde im Vakuum von restlichen flüchtigen Anteilen befreit und filtriert. Über die Bestimmung der Aminzahl konnte ein Umsetzungsgrad von 95 % zum entsprechenden Polyetheramin ermittelt werden. Die titrimetrisch ermittelte Selektivität für die primären Polyetheramine betrug 98 Äquivalent-% bezogen auf den Gesamtamingehalt.

### Beispiel 4

In einen Rührautoklav mit 2 l Volumen wurden 1,0 kg Methylpolyalkylenglykol mit einer mittleren Molmasse von 2000 g/mol, 180 g pulverförmiger Cobalt-Katalysator vom Raney-Typ, dessen Gehaltssumme von Cobalt und Aluminium bezogen auf den wasserfreien Katalysator 94 Masse-% und dessen Kupfergehalt unter 0,2 Masse-% betrug, und 540 ml Ammoniak (flüssig) dosiert. Anschließend wurden 10 bar Wasserstoff aufgepresst und der Autoklav verschlossen. Das Gemisch wurde unter Rühren für 6 Stunden auf 190°C erwärmt. Nach dem Abkühlen wurde langsam entspannt um den Ammoniak und den Wasserstoff zu entfernen. Das verbleibende Reaktionsgemisch wurde im Vakuum von restlichen flüchtigen Anteilen befreit und filtriert. Über die Bestimmung der Aminzahl konnte ein Umsetzungsgrad von 95% zum entsprechenden Polyetheramin ermittelt werden. Die titrimetrisch ermittelte Selektivität für die primären Polyetheramine betrug 97 Äquivalent-% bezogen auf den Gesamtamingehalt.

### Beispiel 5

In einen Schüttelautoklav mit 4,5 I Volumen wurden 750 g Polyalkylenglykol bestehend aus Ethylen- und Propylenglykoleinheiten mit einer mittleren Molmasse von 600 g/mol, 150 g eines Trägerkatalysators, dessen Metallanteil, bezogen auf den trockenen, reduzierten Katalysator ohne das Trägermaterial SiO₂ zu 98 Masse-% aus Cobalt und zu 0,3 Masse-% aus Kupfer bestand, und 800 ml Ammoniak (flüssig) dosiert. Anschließend wurden 10 bar Wasserstoff aufgepresst und der Autoklav verschlossen. Das Gemisch wurde unter Schütteln für 6 Stunden auf 190°C erwärmt. Nach dem Abkühlen wurde langsam entspannt um den Ammoniak und den Wasserstoff zu entfernen. Das verbleibende Reaktionsgemisch wurde im Vakuum von restlichen flüchtigen Anteilen befreit und filtriert. Über die Bestimmung der Aminzahl konnte ein Umsetzungsgrad von 95 % zum entsprechenden Polyetherdiamin ermittelt werden. Die titrimetrisch ermittelte Selektivität für die primären Polyetheramine betrug 96 Äquivalent-% bezogen auf den Gesamtamingehalt.

### Beispiel 6 (Vergleich)

In einen Rührautoklav mit 2 l Volumen wurden 1,0 kg Polyalkylenglykol bestehend aus Ethylen- und Propylenglykoleinheiten mit einer mittleren Molmasse von 4000 g/mol, 200 g eines Trägerkatalysators, dessen Metallanteil, bezogen auf den trockenen, reduzierten Katalysator ohne das Trägermaterial SiO₂ zu 93 Masse-% aus Cobalt und zu 1,2 Masse-% aus Kupfer bestand, und 540 ml Ammoniak (flüssig) dosiert. Anschließend wurden 10 bar Wasserstoff aufgepresst und der Autoklav verschlossen. Das Gemisch wurde unter Rühren für 5 Stunden auf 190°C erwärmt. Nach dem Abkühlen wurde langsam entspannt um den Ammoniak und den Wasserstoff zu entfernen. Das verbleibende Reaktionsgemisch wurde im Vakuum von restlichen flüchtigen Anteilen befreit und filtriert. Über die Bestimmung der Aminzahl konnte ein Umsetzungsgrad von größer 95 % zum entsprechenden Polyetherdiamin ermittelt werden. Die titrimetrisch ermittelte Selektivität für die primären Polyetheramine betrug 98 Äquivalent-% bezogen auf den Gesamtamingehalt.

### Beispiel 7

In einen Schüttelautoklav mit 4,5 I Volumen wurden 1,5 kg Methylpolyalkylenglykol mit einer mittleren Molmasse von 2000 g/mol, 100 g Cobalt-Katalysator vom Raney-Typ, dessen Gehaltssumme an Cobalt und Aluminium bezogen auf den wasserfreien Katalysator 91 Masse-% betrug und 800 ml Ammoniak (flüssig) dosiert. Anschließend wurden 10 bar Wasserstoff aufgepresst und der Autoklav verschlossen. Das Gemisch wurde unter Schütteln für 12 Stunden auf 190°C erwärmt, wobei durch weiteres Aufpressen von Wasserstoff ein Gesamtdruck von 180 bar gehalten wurde. Nach dem Abkühlen wurde langsam entspannt um den Ammoniak und den Wasserstoff zu entfernen. Das verbleibende Reaktionsgemisch wurde im Vakuum von restlichen flüchtigen Anteilen befreit und filtriert. Über die Bestimmung der Aminzahl konnte ein Umsetzungsgrad von 85 % zum entsprechenden Polyetheramin ermittelt werden. Die titrimetrisch ermittelte Selektivität für die primären Polyetheramine betrug 98 Äquivalent-% bezogen auf den Gesamtamingehalt.

### Beispiel 8 (Vergleich)

In einen Rührautoklav mit 2 l Volumen wurden 1,0 kg Fettalkoholoxpropylat mit einer mittleren Molmasse von 310 g/mol, 200 g eines Trägerkatalysators, dessen Metallanteil, bezogen auf den trockenen, reduzierten Katalysator ohne das Trägermaterial zu 94 Masse-% aus Cobalt und zu 0,2 Masse-% aus Kupfer bestand, und 540 ml Ammoniak (flüssig) dosiert. Anschließend wurden 5 bar Wasserstoff aufgepresst und der Autoklav verschlossen. Das Gemisch wurde unter Rühren für 8 Stunden auf 190°C erwärmt. Das entstandene Reaktionswasser verblieb im Rührautoklaven. Nach dem Abkühlen wurde langsam entspannt um den Ammoniak und den Wasserstoff zu entfernen. Das verbleibende Reaktionsgemisch wurde im Vakuum von restlichen flüchtigen Anteilen befreit und filtriert. Über die Bestimmung der Aminzahl konnte ein Umsetzungsgrad von 80 % zum primären Amin ermittelt werden. Ein Gehalt an sekundären und tertiären Aminen war dabei nicht nachweisbar.

### Beispiel 9 (Vergleich)

In einen Rührautoklav mit 2 I Volumen wurden 1,0 kg Fettalkoholoxpropylat mit einer mittleren Molmasse von 310 g/mol, 50 g eines Trägerkatalysators, dessen Metallanteil, bezogen auf den trockenen, reduzierten Katalysator ohne das Trägermaterial zu 94 Masse-% aus Cobalt und zu 0,2 Masse-% aus Kupfer bestand, und 540 ml Ammoniak (flüssig) dosiert. Anschließend wurden 5 bar Wasserstoff aufgepresst und der Autoklav verschlossen. Das Gemisch wurde unter Rühren für 24 Stunden auf 190°C erwärmt. Das entstandene Reaktionswasser verblieb im Autoklaven. Nach dem Abkühlen wurde langsam entspannt um den Ammoniak und den Wasserstoff zu entfernen. Das verbleibende Reaktionsgemisch wurde im Vakuum von restlichen flüchtigen Anteilen befreit und filtriert. Über die Bestimmung der Aminzahl konnte ein Umsetzungsgrad von 75 % zum entsprechenden primären Amin ermittelt werden. Der Gehalt an sekundären und tertiären Aminen war < 1 mol-%.

### Beispiel 10

Ein Festbettreaktor mit einem Durchmesser von 5 cm und einer Länge von 2,5 m wurde mit Tabletten eines Trägerkatalysators, dessen Metallanteil, bezogen auf den trockenen, reduzierten Katalysator ohne das Trägermaterial SiO₂/MgO zu 96 Masse-% aus Cobalt und zu 0,3 Masse-% aus Kupfer bestand, befüllt und der Katalysator unter reduktiven Bedingungen mit Wasserstoff aktiviert. Anschließend wurden bei einer Temperatur von 160°C und einem Gesamtdruck von 100 bar 1 kg/h Fettalkoholoxpropylat mit einer mittleren Molmasse von 310 g/mol, 2,5 kg/h Ammoniak und Wasserstoff in den Reaktor eingespeist. Der Reaktor wurde in Kreisgasfahrweise betrieben und das entstandene Reaktionswasser mit einem Abgasstrom ausgeschleust. Nachdem sich im Reaktor stabile Reaktionsverhältnisse ausgebildet hatten, wurden Proben des Produktaustrags genommen und in den Proben noch gelöster Ammoniak und Wasser im Vakuum entfernt. Über die Bestimmung der Aminzahl und der Hydroxylzahl konnte ein Umsetzungsgrad von 98 % ermittelt werden. Die titrimetrisch ermittelte Selektivität für die primären Amine betrug 98 Äquivalent-% bezogen auf den Gesamtamingehalt.

### Beispiel 11

Es wurde wie in Beispiel 10 beschrieben der Festbettreaktor mit dem Katalysator befüllt und konditioniert. Anschließend wurden bei einer Temperatur von 155°C und einem Gesamtdruck von 90 bar 1 kg/h Fettalkoholoxpropylat mit einer mittleren Molmasse von 310 g/mol, 2,5 kg/h Ammoniak und Wasserstoff in den Reaktor eingespeist. Nachdem sich im Reaktor stabile Reaktionsverhältnisse ausgebildet hatten, wurden Proben des Produktaustrags genommen und in den Proben noch gelöster Ammoniak und Wasser im Vakuum entfernt. Über die Bestimmung der Aminzahl und der Hydroxylzahl konnte ein Umsetzungsgrad von 96 % ermittelt werden. Die titrimetrisch ermittelte Selektivität für die primären Amine betrug 99 Äquivalent-% bezogen auf den Gesamtamingehalt.

### Beispiel 12

Es wurde wie in Beispiel 10 beschrieben der Festbettreaktor mit dem Katalysator befüllt und konditioniert. Anschließend wurden bei einer Temperatur von 160°C und einem Gesamtdruck von 50 bar 1 kg/h Methylpolyalkylenglykol mit einer mittleren Molmasse von 2000 g/mol, 5 kg/h Ammoniak und Wasserstoff in den Reaktor eingespeist. Nachdem sich im Reaktor stabile Reaktionsverhältnisse ausgebildet hatten, wurden Proben des Produktaustrags genommen und in den Proben noch gelöster Ammoniak und Wasser im Vakuum entfernt. Über die Bestimmung der Aminzahl und der Hydroxylzahl konnte ein Umsetzungsgrad von 90 % ermittelt werden. Die titrimetrisch ermittelte Selektivität für die primären Polyetheramine betrug 98 Äquivalent-% bezogen auf den Gesamtamingehalt.

### Beispiel 13 (Vergleichsbeispiel)

In einen Schüttelautoklav mit 4,5 l Volumen, ausgestattet mit einem Katalysatorkorb, wurden 1,0 kg Methylpolyalkylenglykol mit einer mittleren Molmasse von 2000 g/mol, 130 g eines zuvor mit Wasserstoff reduzierten und aktivierten Trägerkatalysators und 600 ml Ammoniak (flüssig) eingebracht. Der reduzierte Trägerkatalysator hatte die Form von etwa 3 mm langen Stäbchen und bestand zu 89 Masse-% aus dem Trägermaterial Aluminiumoxid und zu 11 Masse-% aus Metallen, wobei der Anteil des Cobalts, bezogen auf den Metallgehalt ohne das Trägermaterial Aluminiumoxid, 87 Masse-% und der Anteil des Kupfers, bezogen auf den Metallgehalt ohne das Trägermaterial Aluminiumoxid, 12 Masse-% betrug. Anschließend wurden 30 bar Wasserstoff aufgepresst und der Autoklav verschlossen. Das Gemisch wurde unter Schütteln für 5 Stunden auf 180°C erwärmt. Der Gesamtdruck bei 180°C betrug etwa 160 bar. Nach dem Abkühlen auf 50°C wurde langsam entspannt um den Großteil des Ammoniaks und den Wasserstoff zu entfernen. Das verbleibende Reaktionsgemisch wurde im Vakuum von restlichen flüchtigen Anteilen wie Ammoniak und gebildetes Reaktionswasser befreit und filtriert. Über die Bestimmung der Aminzahl und der Hydroxylzahl konnte ein Umsetzungsgrad von 88 % zum entsprechenden Polyetheramin ermittelt werden. Die titrimetrisch ermittelte Selektivität für die primären Polyetheramine betrug 91 Äquivalent-% bezogen auf den Gesamtamingehalt. Daneben wurden 8 Äquivalent-% sekundäre Polyetheramine und 1 Äquivalent-% tertiäre Polyetheramine bezogen auf den Gesamtamingehalt titrimetrisch nachgewiesen.

## Patentansprüche

1. Verfahren zur kontinuierlichen Herstellung von Polyetheraminen der Formel 1
R²(NR¹R³)ₙ (1)
worin n eine Zahl von 1 bis 20 ist, R² einen organischen Rest darstellt, der zwischen 2 und 600 Oxalkylengruppen enthält, und R¹ und R³ gleich oder verschieden sind und Wasserstoff oder einen organischen Rest mit 1 bis 400 Kohlenstoffatomen darstellen, indem man eine Verbindung der Formel 2 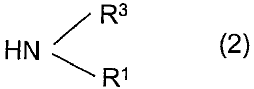 mit einer Verbindung der Formel 3
R²(OH)ₙ (3)
in Gegenwart von Wasserstoff mit einem Katalysator im Festbettreaktor in Verbindung bringt, der metallhaltig ist und dessen Metallgehalt, bezogen auf den trockenen, reduzierten Katalysator ohne eventuell vorhandenes Trägermaterial, entweder zu mindestens 95 Masse-% aus Cobalt oder im Falle der Raney-Katalysatoren zu mindestens 80 Masse-% aus Metallen der Gruppe Cobalt und Aluminium besteht, wobei der Katalysator weniger als 5 Masse-% Kupfer enthält.

2. Verfahren nach Anspruch 1, worin R¹ und R³ Wasserstoff, ein Alkylrest mit 1 bis 50 Kohlenstoffatomen, ein Alkenylrest mit 2 bis 50 Kohlenstoffatomen, ein Arylrest mit 6 bis 50 Kohlenstoffatomen, oder ein Alkylarylrest mit 7 bis 50 Kohlenstoffatomen sind.

3. Verfahren nach Anspruch 1 und/oder 2, worin R¹ und R³ der gleichen Definition wie R² entsprechen.

4. Verfahren nach einem oder mehreren der Ansprüche 1 bis 3, worin R¹ und R³ Aminogruppen enthalten.

5. Verfahren nach einem oder mehreren der Ansprüche 1 bis 4, worin R¹ und R³ der Formel 5 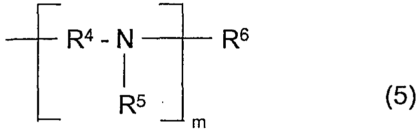 entsprechen, worin R⁴ eine zweiwertige Kohlenwasserstoffgruppe mit 1 bis 50 Kohlenstoffatomen und R⁵ und R⁶ jeweils Wasserstoff oder eine einwertige Kohlenwasserstoffgruppe mit 1 bis 50 Kohlenstoffatomen sein können, wobei jedes von R⁴, R⁵ und R⁶ 1 bis 200 Oxalkylengruppen umfassen kann, und m für eine Zahl von 1 bis 10 steht.

6. Verfahren nach einem oder mehreren der Ansprüche 1 bis 5, worin n für 1, 2, 3 oder 4 steht und worin R² für einen organischen Rest steht, der zwischen 5 und 300 Oxalkylengruppen enthält.

7. Verfahren nach einem oder mehreren der Ansprüche 1 bis 5, worin n für eine ganze Zahl von 5 bis 20 steht und worin R² für einen organischen Rest steht, der 2 bis 300 Oxalkylengruppen enthält.

8. Verfahren nach Anspruch 1, worin R¹ und R³ Wasserstoff bedeuten.

9. Verfahren nach einem oder mehreren der Ansprüche 1 bis 8, worin der Katalysator vom Typ Raney-Cobalt mindestens 90 Masse-% Metalle bestehend aus der Gruppe Cobalt und Aluminium enthält und die Masse-% bezogen sind auf den trockenen Katalysator.

10. Verfahren nach einem oder mehreren der Ansprüche 1 bis 9, worin der Katalysator zwischen weniger als 5 und 0,1 Masse-% Kupfer enthält.

11. Verfahren nach einem oder mehreren der Ansprüche 1 bis 10, worin der Katalysator weniger als 4 Masse-% Kupfer enthält.

12. Verfahren nach einem oder mehreren der Ansprüche 1 bis 11, worin n gleich eins oder zwei ist.

13. Verfahren nach einem oder mehreren der Ansprüche 1 bis 12, worin die Reaktionstemperatur 50 bis 250°C beträgt.

14. Verfahren nach einem oder mehreren der Ansprüche 1 bis 13, worin der Gesamtdruck 1 bis 300 bar beträgt.

## Claims

1. A process for the continuous preparation of polyetheramines of the formula 1
R²(NR¹R³)ₙ (1)
in which n is a number from 1 to 20, R² is an organic radical which comprises between 2 and 600 oxyalkylene groups, and R¹ and R³ are the same or different and are each hydrogen or an organic radical having from 1 to 400 carbon atoms, by combining a compound of the formula 2 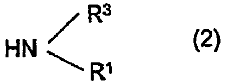 with a compound of the formula 3
R²(OH)ₙ (3)
in the presence of hydrogen in a fixed bed reactor with a catalyst which is metallic and whose metal content, based on the dry, reduced catalyst excluding any support material present, either consists of cobalt to an extent of at least 95% by mass or, in the case of the Raney catalysts, consists of metals from the group of cobalt and aluminum to an extent of at least 80% by mass, and which contains less than 5% by mass of copper.

2. The process as claimed in claim 1, in which R¹ and R³ are each hydrogen, an alkyl radical having from 1 to 50 carbon atoms, an alkylene radical having from 2 to 50 carbon atoms, an aryl radical having from 6 to 50 carbon atoms or an alkylaryl radical having from 7 to 50 carbon atoms.

3. The process as claimed in claim 1 and/or 2, in which R¹ and R³ are each as defined for R².

4. The process as claimed in one or more of claims 1 to 3, in which R¹ and R³ each contain amino groups.

5. The process as claimed in one or more of claims 1 to 4, in which R¹ and R³ each correspond to the formula 5 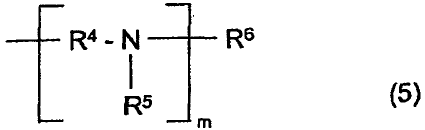 in which R⁴ is a divalent hydrocarbon group having from 1 to 50 carbon atoms and R⁵ and R⁶ may each be hydrogen or a monovalent hydrocarbon group having from 1 to 50 carbon atoms, where each of R⁴, R⁵ and R⁶ may comprise from 1 to 200 oxyalkylene groups, and m is from 1 to 10.

6. The process as claimed in one or more of claims 1 to 5, in which n is 1, 2, 3 or 4, and in which R² is an organic radical which has between 5 and 300 oxyalkylene groups.

7. The process as claimed in one or more of claims 1 to 5, in which n is an integer from 5 to 20, and in which R² is an organic radical which has from 2 to 300 oxyalkylene groups.

8. The process as claimed in claim 1, in which R¹ and R³ are each hydrogen.

9. The process as claimed in one or more of claims 1 to 8, in which the catalyst of the Raney cobalt type contains at least 90% by mass of metals consisting of the group of cobalt and aluminum and the percentages by mass are based on the dry catalyst.

10. The process as claimed in one or more of claims 1 to 9, in which the catalyst contains between less than 5 and 0.1% by mass of copper.

11. The process as claimed in one or more of claims 1 to 10, in which the catalyst contains less than 4% by mass of copper.

12. The process as claimed in one or more of claims 1 to 11, in which n is one or two.

13. The process as claimed in one or more of claims 1 to 12, in which the reaction temperature is from 50 to 250°C.

14. The process as claimed in one or more of claims 1 to 13, in which the overall pressure is from 1 to 300 bar.

## Revendications

1. Procédé pour la préparation continue de polyétheramines de formule 1,
R²(NR¹R³)ₙ (1)
dans laquelle n est un nombre de 1 à 20, R² représente un radical organique, qui contient entre 2 et 600 groupes oxyalkylène, et R¹ et R³ sont identiques ou différents et représentent hydrogène ou un radical organique comprenant 1 à 400 atomes de carbone, selon lequel on rassemble un composé de formule 2 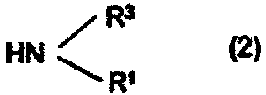 avec un composé de formule 3
R²(OH)ₙ (3)
en présence d'hydrogène dans un réacteur à lit fixe avec un catalyseur, qui contient du métal et dont la teneur en métal par rapport au catalyseur sec, réduit, sans matériau support éventuellement présent, est constituée, soit à raison d'au moins 95% en masse de cobalt, soit, dans le cas de catalyseurs de Raney, à raison d'au moins 80% en masse de métaux du groupe formé par le cobalt et l'aluminium, le catalyseur contenant moins de 5% en masse de cuivre.

2. Procédé selon la revendication 1, dans lequel R¹ et R³ représentent hydrogène, un radical alkyle comprenant 1 à 50 atomes de carbone, un radical alcényle comprenant 2 à 50 atomes de carbone, un radical aryle comprenant 6 à 50 atomes de carbone ou un radical alkylaryle comprenant 7 à 50 atomes de carbone.

3. Procédé selon la revendication 1 et/ou 2, dans lequel R¹ et R³ ont la même signification que R².

4. Procédé selon l'une ou plusieurs des revendications 1 à 3, dans lequel R¹ et R³ contiennent des groupes amino.

5. Procédé selon l'une ou plusieurs des revendications 1 à 4, dans lequel R¹ et R³ correspondent à la formule 5 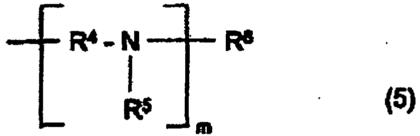 dans laquelle R⁴ peut représenter un groupe hydrocarboné divalent comprenant 1 à 50 atomes de carbone et R⁵ et R⁶ peuvent représenter à chaque fois hydrogène ou un groupe hydrocarboné monovalent comprenant 1 à 50 atomes de carbone, chacun parmi R⁴, R⁵ et R⁶ pouvant comprendre 1 à 200 groupes oxyalkylène et m représente un nombre de 1 à 10.

6. Procédé selon l'une ou plusieurs des revendications 1 à 5, dans lequel n représente 1, 2, 3 ou 4 et dans lequel R₂ représente un radical organique qui contient entre 5 et 300 groupes oxyalkylène.

7. Procédé selon l'une ou plusieurs des revendications 1 à 5, dans lequel n représente un nombre entier de 5 à 20 et dans lequel R₂ représente un radical organique qui contient 2 à 300 groupes oxyalkylène.

8. Procédé selon la revendication 1, dans lequel R₁ et R₃ signifient hydrogène.

9. Procédé selon l'une ou plusieurs des revendications 1 à 8, dans lequel le catalyseur du type cobalt de Raney contient au moins 90% en masse de métaux constitués par le groupe formé par le cobalt et l'aluminium et les % en masse se rapportent au catalyseur sec

10. Procédé selon l'une ou plusieurs des revendications 1 à 9, dans lequel le catalyseur contient entre moins de 5 et 0,1% en masse de cuivre.

11. Procédé selon l'une ou plusieurs des revendications 1 à 10, dans lequel le catalyseur contient moins de 4% en masse de cuivre.

12. Procédé selon l'une ou plusieurs des revendications 1 à 11, dans lequel le n vaut un ou deux.

13. Procédé selon l'une ou plusieurs des revendications 1 à 12, dans lequel la température de réaction est de 50 à 250°C.

14. Procédé selon l'une ou plusieurs des revendications 1 à 13, dans lequel la pression totale est de 1 à 300 bars.
